# EUROPEAN PATENT APPLICATION

(11) **EP 4 530 347 A1**
(43) Date of publication of application: **02.04.2025**
(21) Application number: 23811825.1
(22) Date of filing: 23.05.2023
(51) Int. Cl.: C12N 9/14

(54) **LIQUID ENZYME PREPARATION**

(30) Priority: 23.05.2022 JP 2022083652
(71) Applicant: Amano Enzyme Inc., Nagoya-shi Aichi 460-8630 (JP)
(72) Inventor: HIURA, Keita, Kakamigahara-shi, Gifu 509-0109 (JP); YACHI, Hiroyuki, Kakamigahara-shi, Gifu 509-0109 (JP); MITO, Koji, Kitanagoya-shi, Aichi 481-8533 (JP)
(74) Representative: Müller-Boré & Partner Patentanwälte PartG mbB
(86) International application number: PCT/JP2023/019217
(87) International publication number: WO 2023/228953

(57) **Abstract**

The present invention provides a liquid enzyme preparation of an enzyme having excellent activity stability. Stability of enzymatic activity is improved by accommodating a liquid enzyme preparation that includes an enzyme into a container the internal space of which has been replaced by an inert gas, or by adding, to a liquid enzyme preparation containing a protein deamidase, an additive selected from the group consisting of sugar alcohols, glycerin, ethanol, sulfites, lecithins, citrates, polyphosphates, tartrates, phytic acid, metaphosphoric acid, sodium chloride, tocotrienols, methionine, pectins, beet pigment, capsicum pigment, rosemary extract, tocopherols, tea extract, and butylated hydroxyanisole.

## Description

### Technical Field

The present invention relates to a liquid enzyme preparation. More specifically, the present invention relates to a liquid enzyme preparation having excellent enzyme activity stability.

### Background Art

As an enzyme to deamidate a γ-amide group and a β-amide group of a glutamine residue and an asparagine residue in a protein, a protein glutaminase derived from Chryseobacterium gleum JCM2410 strain and a protein glutaminase derived from Chryseobacterium proteolyticum 9670 strain are known as described in PTL 1 and the like. Enzyme preparation products of such protein deamidases are currently only commercially available in powder form.

As for the enzyme preparation, in general, liquid form is more desirable than powder form in consideration of dusting and handling. For this reason, techniques to formulate an enzyme preparation in a liquid form have been studied for various enzymes. For example, PTL 2 proposes a liquid enzyme preparation in which at least one enzyme to crosslink and/or modify a milk protein is contained in a polyol-water suspension containing 25 to 100% (w/w) polyol and having a pH value in the range of 4.4 to 5.1. Specifically, PTL 2 finds that the stability of a transglutaminase is low at pH 5.2, and based on this finding, discloses that the stability of a transglutaminase preparation in a glycerol-water suspension at pH 4.4 to 4.8 and a transglutaminase preparation in a sorbitol-water suspension at pH 4.6 are improved, and in addition, also discloses a tyrosinase preparation in a glycerol-water suspension at pH 4.6 and a protein glutaminase preparation in a glycerol-water suspension at pH 4.6.

In addition, PTL 3 describes that a useful protein composition containing a useful protein, a solvent, and a compound having a thiol structure is subjected to bubbling with an asphyxiating gas such as nitrogen to set the concentration of dissolved oxygen to 3 mg/L or less, and thereby a useful protein composition such as a stable interferon can be produced.

### Citation List

### Patent Literature

PTL 1: International Publication No. WO 2010/029685
PTL 2: International Publication No. WO 2013/064736
PTL 3: Japanese Patent Laid-open Publication No. 2013-049650

### Summary of Invention

### Technical Problem

In PTL 2, the stability of a liquid enzyme preparation of a transglutaminase preparation has been studied, while a liquid enzyme preparation of a protein deamidase has been only disclosed as a preparation formulation, and the stability of this enzyme has not been sufficiently studied. In PTL 3, while the antiviral activity of an aqueous preparation containing dog interferon-γ is examined, there is no mention to a liquid enzyme preparation of a protein deamidase, and naturally no examination has not been made.

Therefore, an object of the present invention is to provide a liquid enzyme preparation having excellent activity stability of an enzyme such as a protein deamidase. Solution to Problem

As a result of intensive studies, the present inventor has found that, when a liquid enzyme preparation containing an enzyme is packed in a container whose internal space is replaced with an inert gas, the activity stability of the enzyme is remarkably improved. This effect can be obtained even when the amount of dissolved oxygen in the liquid preparation exceeds 3 mg/L, at which PTL 3 shows that the stability of useful proteins is decreased. Therefore, it was completely unexpected that a liquid enzyme preparation containing an enzyme can obtain such an effect only by replacing the internal space with an inert gas regardless of the amount of dissolved oxygen.

Furthermore, the present inventor has found that the activity stability of a protein deamidase is improved also by adding a predetermined additive to a liquid enzyme preparation containing a protein deamidase. In view of the fact that this effect could not be obtained when many other components known as an antioxidant, such as ascorbic acid, were used, it has become clear that this effect is a specific effect obtained by using the predetermined additive.

The present invention has been completed based on these findings. That is, the present invention includes the following inventions.

Item 1. A liquid enzyme preparation including an enzyme, wherein the liquid enzyme preparation is packed in a container whose internal space is replaced with an inert gas (hereinafter, also referred to as "first liquid enzyme preparation").

Item 2. The liquid enzyme preparation according to item 1, in which the enzyme is a protein deamidase or a transglutaminase.

Item 3. The liquid enzyme preparation according to item 1 or 2, in which the enzyme activity is 0.1 to 10,000 U/ml.

Item 4. The liquid enzyme preparation according to any one of items 1 to 3, including an additive selected from the group consisting of sugar alcohol, glycerin, ethanol, sulfite, lecithin, citrate, polyphosphate, tartrate, phytic acid, metaphosphoric acid, sodium chloride, tocotrienol, methionine, pectin, beet dye, paprika dye, rosemary extract, tocopherol, tea extract, and butylhydroxyanisole.

Item 5. A liquid enzyme preparation including: a protein deamidase; and an additive selected from the group consisting of sugar alcohol, glycerin, ethanol, sulfite, lecithin, citrate, polyphosphate, tartrate, phytic acid, metaphosphoric acid, sodium chloride, tocotrienol, methionine, pectin, beet dye, paprika dye, rosemary extract, tocopherol, tea extract, and butylhydroxyanisole (hereinafter, also referred to as "second liquid enzyme preparation").

Item 6. The liquid enzyme preparation according to item 4 or 5, in which the additive is contained in a total amount of 5 w/v% or more.

Item 7. The liquid enzyme preparation according to any one of items 4 to 6, in which the additive is contained in a total amount of 80 w/v% or less.

Item 8. The liquid enzyme preparation according to any one of items 1 to 7, having a pH of 5.5 or more.

Item 9. The liquid enzyme preparation according to any one of items 1 to 8, having a pH of 8.2 or less.

Item 10. The liquid enzyme preparation according to any one of items 5 to 9, in which the protein deamidase activity is 0.1 to 10,000 U/ml.

Item 11. The liquid enzyme preparation according to any one of items 2 to 10, in which the protein deamidase is derived from Chryseobacterium proteolyticum.

Item 12. An enzyme activity stabilization method for a liquid enzyme preparation, the method including: packing a liquid enzyme preparation containing an enzyme in a container whose internal space is replaced with an inert gas.

Item 13. A protein deamidase activity stabilizer for a liquid enzyme preparation containing a protein deamidase, the protein deamidase activity stabilizer including an additive selected from the group consisting of sugar alcohol, glycerin, ethanol, sulfite, lecithin, citrate, polyphosphate, tartrate, phytic acid, metaphosphoric acid, sodium chloride, tocotrienol, methionine, pectin, beet dye, paprika dye, rosemary extract, tocopherol, tea extract, and butylhydroxyanisole.

### Advantageous Effects of Invention

According to the present invention, a liquid enzyme preparation having excellent activity stability of an enzyme is provided.

### Description of Embodiments

### [1. Liquid enzyme preparation]

The first liquid enzyme preparation of the present invention includes an enzyme, wherein the liquid enzyme preparation is packed in a container whose internal space is replaced with an inert gas. The second liquid enzyme preparation of the present invention includes: a protein deamidase; and an additive selected from the group consisting of sugar alcohol, glycerin, ethanol, sulfite, lecithin, citrate, polyphosphate, tartrate, phytic acid, metaphosphoric acid, sodium chloride, tocotrienol, methionine, pectin, beet dye, paprika dye, rosemary extract, tocopherol, tea extract, and butylhydroxyanisole. Hereinafter, the first liquid enzyme preparation and the second liquid enzyme preparation (The first liquid enzyme preparation and the second liquid enzyme preparation are also collectively referred to simply as liquid enzyme preparation.) of the present invention will be described in detail.

### [1-1. Enzyme]

The enzyme contained in the first liquid enzyme preparation of the present invention is not particularly limited. Preferable examples of the enzyme include a protein deamidase and a transglutaminase, and particularly preferable examples thereof include a protein deamidase. The enzyme contained in the second liquid enzyme preparation of the present invention is a protein deamidase.

### [Protein deamidase]

The protein deamidase is an enzyme that exhibits an action of decomposing an amide group-containing side chain of a protein without cleavage of a peptide bond and crosslinking of a protein, and the type, origin, and the like thereof are not particularly limited. In addition, as long as the above action is main activity, the protein deamidase may further have an action of decomposing an amide group-containing side chain of a protein accompanied by cleavage of a peptide bond and crosslinking of a protein. Examples of the protein deamidase include an enzyme that deamides a glutamine residue in a protein and converts the residue into glutamic acid (for example, protein glutaminase) and an enzyme that deamides an asparagine residue in a protein and converts the residue into aspartic acid (for example, protein asparaginase). More specific examples of the protein deamidase include: a protein deamidase derived from the genus Chryseobacterium, Flavobacterium, Empedobacter, Sphingobacterium, Aureobacterium, or Myroides disclosed in JP 2000-50887 A, JP 2001-218590 A, and WO 2006/075772 A1; a protein deamidase derived from the genus Luteimicrobium, Agromyces, Microbacterium, or Leifsonia disclosed in WO 2015/133590; and commercially available products of a protein glutaminase derived from the genus Chryseobacterium. These protein deamidases may be used singly or in combination of two or more kinds thereof.

Among these protein deamidases, a protein deamidase derived from the genus Chryseobacterium is preferable, a protein glutaminase derived from the genus Chryseobacterium is more preferable, and a protein glutaminase derived from the species Chryseobacterium proteolyticum is still more preferable from the viewpoint of obtaining a liquid enzyme preparation further excellent in activity stability.

The protein deamidase can be prepared from a culture solution of a microorganism from which the protein deamidase is derived. Specific examples of the preparation method include a method of recovering a protein deamidase from the above culture solution of a microorganism or a bacterial cell. For example, in the case of using a microorganism that secrets a protein deamidase, bacterial cells are recovered from the culture solution in advance by filtration, centrifugation, or the like as necessary, and then, an enzyme can be separated and/or purified. In addition, in the case of using a microorganism that does not secret a protein deamidase, bacterial cells are recovered from the culture solution in advance as necessary, the recovered bacterial cells are disrupted by pressurization treatment, ultrasonic treatment, or the like to expose an enzyme, and the exposed enzyme can be separated and/or purified. As the method of separating and/or purifying an enzyme, a known method of separating and/or purifying a protein can be used without particular limitation. Examples thereof include a centrifugal separation method, a UF concentration method, a salting-out method, various chromatography methods using an ion exchange resin and the like.

In the liquid enzyme preparation of the present invention, the content of the protein deamidase is not particularly limited as long as it is possible to effectively exert the action of the protein deamidase in use of the liquid enzyme preparation. Examples thereof include 0.1 to 10,000 U/mL, 1 to 5,000 U/mL, 10 to 3,000 U/mL, or 50 to 2,000 U/mL, preferably 100 to 1,500 U/mL, more preferably 200 to 1,000 U/mL, still more preferably 300 to 900 U/mL, or 500 to 800 U/mL, and still more preferably 600 to 700 U/mL.

For the protein deamidase activity, the enzyme amount to liberate 1 µmol of ammonia per minute using benzyloxycarbonyl-L-glutaminylglycine (Z-Gln-Gly) as a substrate is defined as 1 unit (1 U).

### [Transglutaminase]

The transglutaminase is an enzyme (EC2.3.2.13) having an activity of crosslinking a protein by catalyzing an acyl transfer reaction between a γ-carboxamide group of a glutamine residue and an ε-amino group of a lysine residue in a protein, and the type, origin, and the like thereof are not particularly limited. The transglutaminase includes both calcium-dependent, which requires calcium for activity expression, and calcium-independent, which requires no calcium for activity expression.

Specific examples of the transglutaminase used in the present invention are not particularly limited. Specific examples of the transglutaminase include: a microorganism-derived transglutaminase, specifically, a transglutaminase derived from the genus Streptomyces such as Streptomyces ladakanum, Streptomyces cinnamoneus, Streptomyces griseocarneus, Streptomyces lavendulae, or Streptomyces lydicus (See, for example, JP S64-27471 A.), a transglutaminase derived from the genus Kutzneria such as Kutzneria albida (See, for example, JP 2020-195397 A.), and a transglutaminase derived from the genus Longimycelium such as Longimycelium tulufanense; a mammal-derived transglutaminase (See, for example, JP H01-50382 B.); a fish-derived transglutaminase (See, for example, JP H06-113844 A.); a recombinant transglutaminase obtained using recombinant DNA technology (See, for example, JP H05-199883 A and JP 2004-97099 A.); and a transglutaminase having such a structure that a pro-sequence peptide of transglutaminase is bound to matured transglutaminase (See, for example, WO 2009/101762 A1). These transglutaminases may be used alone, or may be used in combination of two or more thereof.

Among these transglutaminases, from the viewpoint of further enhancing the stabilization effect, a microorganism-derived transglutaminase is preferable, and a transglutaminase derived from the genus Streptomyces is more preferable, and a transglutaminase derived from the species Streptomyces mobaraensis is still more preferable.

In the first liquid enzyme preparation of the present invention, the content of the transglutaminase is not particularly limited as long as it is possible to effectively exert the action of the transglutaminase in use of the liquid enzyme preparation. Examples thereof include 1 to 100,000 U/mL, preferably 10 to 50,000 U/mL, more preferably 90 to 10,000 U/mL, still more preferably 180 to 5,000 U/mL, 280 to 3,000 U/mL, or 380 to 1,500 U/mL, and still more preferably 480 to 1,000 U/mL, 490 to 800 U/mL, or 500 to 700 U/mL.

For the activity value of the transglutaminase, the enzyme activity to produce 1 µmol of hydroxamic acid per minute using benzyloxycarbonyl-L-glutaminylglycine and hydroxylamine as a substrate is defined as 1 unit (U).

### [1-2. Predetermined additive]

The second liquid enzyme preparation of the present invention contains a predetermined additive from the viewpoint of obtaining a liquid enzyme preparation further excellent in activity stability. The first liquid enzyme preparation of the present invention can also contain the predetermined additive.

In the liquid enzyme preparation of the present invention, the predetermined additive is selected from the group consisting of sugar alcohol, glycerin, ethanol, sulfite, lecithin, citrate, polyphosphate, tartrate, phytic acid, metaphosphoric acid, sodium chloride, tocotrienol, methionine, pectin, beet dye, paprika dye, rosemary extract, tocopherol, tea extract, and butylhydroxyanisole.

Examples of the sugar alcohol include sorbitol, xylitol, and maltitol. These sugar alcohols may be used singly or in combination of two or more kinds thereof.

Examples of the sulfite, citrate, polyphosphate, and tartrate described above preferably include alkali metal salts such as potassium salts and sodium salts, and more preferably include sodium salts.

Examples of the lecithin include: natural lecithin such as soybean lecithin, sunflower lecithin, rapeseed lecithin, and egg yolk lecithin; enzyme-treated lecithin (mainly composed of phosphatidylglycerol obtained from the natural lecithin); enzyme-degraded lecithin (mainly composed of phosphatidic acid and lysolecithin obtained from the natural lecithin); and fractional lecithin (mainly composed of sphingomyelin, phosphatidylinositol, phosphatidylethanolamine, and phosphatidylcholine obtained from the natural lecithin). These lecithins may be used singly or in combination of two or more kinds thereof. Among these lecithins, soybean lecithin, sunflower lecithin, and rapeseed lecithin are preferable from the viewpoint of further improving the enzyme activity stability in the liquid enzyme preparation that is in contact with a gas phase not substituted with an inert gas.

Examples of the tocotrienol include α-tocotrienol, γ-tocotrienol, and δ-tocotrienol. These tocotrienols may be used singly or in combination of two or more kinds thereof. Among these tocotrienols, a mixture of α-tocotrienol, γ-tocotrienol, and δ-tocotrienol is preferable from the viewpoint of further improving the enzyme activity stability in the liquid enzyme preparation that is in contact with a gas phase not substituted with an inert gas. For blending the tocotrienol, a rice oil extract represented by Oryza tocotrienol can be preferably used from the viewpoint of further improving the enzyme activity stability in the liquid enzyme preparation that is in contact with a gas phase not substituted with an inert gas.

The pectin is not particularly limited. Specific examples of the pectin include pectin derived from sugar beet, citrus fruits, apple, and the like, and pectin derived from sugar beet is preferable from the viewpoint of further improving the enzyme activity stability in the liquid enzyme preparation that is in contact with a gas phase not substituted with an inert gas.

In the liquid enzyme preparation of the present invention, the content of the predetermined additive is not particularly limited, and is, for example, 5 w/v% or more, preferably 8 w/v% or more, 15 w/v% or more, 20 w/v% or more, 25 w/v% or more, 30 w/v% or more, 35 w/v% or more, 40 w/v% or more, 50 w/v% or more, 60 w/v% or more, or 70 w/v% or more, in total. The content of the predetermined additive is not particularly limited even in its upper limit, and is, for example, 80 w/v% or less, 75 w/v% or less, 65 w/v% or less, 55 w/v% or less, 45 w/v% or less, 35 w/v% or less, 25 w/v% or less, 20 w/v% or less, or 15 w/v% or less, in total. In the liquid enzyme preparation of the present invention, the content of the predetermined additive per 1 U of the enzyme is, for example, 0.075 mg or more, preferably 0.1 mg or more, 0.2 mg or more, 0.3 mg or more, 0.4 mg or more, 0.45 mg or more, 0.5 mg or more, 0.6 mg or more, 0.75 mg or more, 0.9 mg or more, or 1 mg or more, in total. In the liquid enzyme preparation of the present invention, the content of the predetermined additive per 1 U of the enzyme is not particularly limited even in its upper limit, and is 5 mg or less, 4 mg or less, 3 mg or less, 2 mg or less, 1.5 mg or less, 1.2 mg or less, 1.1 mg or less, 1 mg or less, 0.85 mg or less, 0.7 mg or less, 0.55 mg or less, 0.4 mg or less, 0.35 mg or less, or 0.3 mg or less, in total.

When any component selected from the group consisting of sugar alcohol and glycerin is contained as the predetermined additive, the content of each component is, for example, 10 w/v% or more, 15 w/v% or more, 20 w/v% or more, 25 w/v% or more, or 30 w/v% or more, preferably 35 w/v% or more, or 40 w/v% or more, more preferably 50 w/v% or more, still more preferably 60 w/v% or more, still more preferably 70 w/v% or more. In this case, the upper limit of the content range of each component is not particularly limited, and examples thereof include 80 w/v% or less, 75 w/v% or less, 65 w/v% or less, 55 w/v% or less, 45 w/v% or less, 35 w/v% or less, 25 w/v% or less, 20 w/v% or less, or 15 w/v% or less. In the liquid enzyme preparation of the present invention, the content of each of the components selected from the group consisting of sugar alcohol and glycerin per 1 U of the enzyme is, for example, 0.15 mg or more, 0.2 mg or more, 0.3 mg or more, 0.4 mg or more, or 0.45 mg or more, preferably 0.5 mg or more, or 0.6 mg or more, more preferably 0.75 mg or more, still more preferably 0.9 mg or more, even more preferably 1 mg or more. In the liquid enzyme preparation of the present invention, the upper limit of the content range of each of the components selected from the group consisting of sugar alcohol and glycerin per 1 U of the enzyme is not particularly limited, and examples thereof include 5 mg or less, 4 mg or less, 3 mg or less, 2 mg or less, 1.5 mg or less, 1.2 mg or less, 1.1 mg or less, 1 mg or less, 0.85 mg or less, 0.7 mg or less, 0.55 mg or less, 0.4 mg or less, 0.35 mg or less, or 0.3 mg or less. When two or more components selected from the group consisting of sugar alcohol and glycerin are contained in combination, the components can be contained in the liquid enzyme preparation of the present invention so that the total amount thereof is within the above-described content (amount indicated as the content of each of the components selected from the group consisting of sugar alcohol and glycerin).

When the predetermined additive includes sulfite, lecithin, polyphosphate, phytic acid, metaphosphoric acid, tocotrienol, methionine, pectin, beet dye, paprika dye, rosemary extract, tocopherol, tea extract, and/or butylhydroxyanisole, the content of each of these components is, for example, 0.01 w/v% or more, preferably 0.03 w/v% or more, more preferably 0.05 w/v% or more, 0.1 w/v% or more, 0.2 w/v% or more, 0.4 w/v% or more, 0.6 w/v% or more, 0.8 w/v% or more, 1 w/v% or more, 1.5 w/v% or more, or 2 w/v% or more. In this case, the upper limit of the content range of each of these components is not particularly limited, and examples thereof include 12 w/v% or less, preferably 10 w/v% or less, more preferably 6 w/v% or less, 4 w/v% or less, 3 w/v% or less, 2 w/v% or less, or 1 w/v% or less. In the liquid enzyme preparation of the present invention, the content of each of these components per 1 U of the enzyme is, for example, 0.00005 mg or more, preferably 0.0001 mg or more, more preferably 0.0005 mg or more, still more preferably 0.00075 mg or more, still more preferably 0.001 mg or more, or 0.0012 mg or more. In the liquid enzyme preparation of the present invention, the upper limit of the content range of each of these components per 1 U of the enzyme is not particularly limited, and examples thereof include 2.5 mg or less, 2 mg or less, 1.5 mg or less, 1 mg or less, 0.75 mg or less, 0.5 mg or less, 0.25 mg or less, 0.2 mg or less, or 0.175 mg or less. In addition, when a combination of two or more of sulfite, lecithin, polyphosphate, phytic acid, metaphosphoric acid, tocotrienol, methionine, pectin, beet dye, paprika dye, rosemary extract, tocopherol, tea extract, and butylhydroxyanisole is contained, the components can be contained in the liquid enzyme preparation of the present invention so that the total amount thereof is within the above-described content (amount shown as the content of each thereof).

When ethanol, citrate, tartrate, and/or sodium chloride are contained as the predetermined additive, the content of each of these components is, for example, 0.5 w/v% or more, preferably 0.8 w/v% or more, more preferably 2.5 w/v% or more, still more preferably 4.5 w/v% or more, 6 w/v% or more, 8 w/v% or more, 9 w/v% or more, 12 w/v% or more, or 14 w/v% or more. In this case, the upper limit of the content range of each of these components is not particularly limited, and examples thereof include 20 w/v% or less, 18 w/v% or less, 16 w/v% or less, 13 w/v% or less, 11 w/v% or less, 9 w/v% or less, 7 w/v% or less, or 6 w/v% or less. In the liquid enzyme preparation of the present invention, the content of each of these components per 1 U of the enzyme is, for example, 0.0075 mg or more, preferably 0.012 mg or more, more preferably 0.037 mg or more, still more preferably 0.068 mg or more, 0.09 mg or more, 0.12 mg or more, 0.14 mg or more, 0.18 mg or more, or 0.2 mg or more. In the liquid enzyme preparation of the present invention, the upper limit of the content range of each of these components per 1 U of the enzyme is not particularly limited, and examples thereof include 0.3 mg or less, 0.27 mg or less, 0.24 mg or less, 0.2 mg or less, 0.16 mg or less, 0.14 mg or less, 0.1 mg or less, or 0.09 mg or less. In addition, when a combination of two or more of ethanol, citrate, tartrate, and sodium chloride is contained, the components can be contained in the liquid enzyme preparation of the present invention so that the total amount thereof is within the above-described content (amount shown as the content of each thereof).

### [1-3. Other components]

The liquid enzyme preparation of the present invention optionally contains other components in addition to the above-described enzyme and predetermined additives as long as the effect of the present invention is not adversely affected. Examples of the other components include buffers, other enzymes, and other additives other than the predetermined additives.

Examples of other enzymes include amylase (α-amylase, β-amylase, glucoamylase), glucosidase (α-glucosidase, β-glucosidase), galactosidase (α-galactosidase, β-galactosidase), peptidase (leucine peptidase, aminopeptidase), lipase, esterase, cellulase, phosphatase (acid phosphatase, alkaline phosphatase), nuclease, deaminase, oxidase, dehydrogenase, glutaminase, pectinase, catalase, dextranase, transglutaminase, and pullulanase. These other enzymes may be contained alone or in combination of two or more thereof.

Examples of other additives include buffers, suspending agents, and physiological saline. Examples of the buffer include a phosphate buffer, an acetate buffer, and a citrate buffer. These other additives may be contained alone or in combination of two or more thereof.

### [1-4. Preparation form and the like]

The liquid enzyme preparation of the present invention is a liquid preparation. The liquid enzyme preparation of the present invention can be prepared by: adding, into water, a predetermined amount of the enzyme, the predetermined additive, and other components to be blended as necessary, or performing separation and/or purification to remove unnecessary components such as contaminant polymers from a culture solution of an enzyme producing strain and subsequently adding the predetermined additive and other components to be blended as necessary; and further sterilizing as necessary.

The pH of the liquid enzyme preparation of the present invention (referring to pH at 25°C; The same applies hereinafter.) is not particularly limited, and is, for example, 5.5 or more, preferably 5.8 or more. In the liquid enzyme preparation of the present invention, the upper limit of the pH range is not particularly limited, and is preferably 8.2 or less, more preferably 7.5 or less, still more preferably 7 or less, and still more preferably 6.5 or less.

### [1-5. Amount of dissolved oxygen]

The amount of dissolved oxygen contained in the liquid enzyme preparation of the present invention is not particularly limited. In particular, the first liquid enzyme preparation of the present invention is excellent in enzyme activity stability. Therefore, the activity-stabilizing effect can be effectively obtained even when the amount of dissolved oxygen contained in the first liquid enzyme preparation is not actively reduced. From such a viewpoint, a suitable example of the amount of dissolved oxygen in the first liquid enzyme preparation is more than 3 mg/L, preferably more than 4 mg/L, more preferably 4.5 mg/L or more, and still more preferably 5 mg/L or more. The upper limit of the range of dissolved oxygen amount in the first liquid enzyme preparation is not particularly limited, and is, for example, 8 mg/L or less, preferably 7 mg/L or less, more preferably 6 mg/L or less, and still more preferably 5.5 mg/L or less. The amount of dissolved oxygen in the first liquid enzyme preparation can also be applied to the amount of dissolved oxygen contained in the second liquid enzyme preparation of the present invention. The liquid enzyme preparation of the present invention does not exclude the case where the amount of dissolved oxygen is 3 mg/L or less.

### [1-6. Packing in container]

The liquid enzyme preparation of the present invention is appropriately packed in a container capable of packing a liquid. The first liquid enzyme preparation of the present invention is packed in a container whose internal space is replaced with an inert gas. The inert gas is not particularly limited, and examples thereof include a nitrogen gas and a rare gas (helium gas, argon gas, etc.). The method for replacing the inner space of the container with an inert gas is not particularly limited, and examples thereof include a method of directly adding a gaseous inert gas or a liquefied inert gas into the container.

The first liquid enzyme preparation of the present invention, packed in a container whose internal space is replaced with an inert gas, can improve the enzyme activity stability, without bubbling an inert gas into the liquid enzyme preparation. Therefore, in a preferred embodiment of the first liquid enzyme preparation of the present invention, an inert gas is not bubbled. The first liquid enzyme preparation of the present invention does not exclude a form in which an inert gas is bubbled.

In the first liquid enzyme preparation of the present invention contained in the container, the oxygen concentration in the container (that is, in a gas phase replaced with an inert gas in the container) is, for example, 5 v/v% or less, preferably 3 v/v% or less, more preferably 2 v/v% or less, 1.5 v/v% or less, 1.3 v/v% or less, or 1 v/v% or less. The oxygen concentration in the container is not particularly limited in its lower limit, and is, for example, 0.01 v/v% or more, 0.05 v/v% or more, 0.1 v/v% or more, 0.4 v/v% or more, 0.6 v/v% or more, 0.8 v/v% or more, or 1 v/v% or more.

The first liquid enzyme preparation of the present invention is packed in a container whose internal space is replaced with an inert gas. Thereby, as described above, an excellent enzyme activity-stabilizing effect is exhibited. Furthermore, when the first liquid enzyme preparation is opened at the time of use and brought into contact with a gas phase not substituted with an inert gas, the first liquid enzyme preparation becomes the form of the second liquid enzyme preparation, and exhibits an enzyme activity-stabilizing effect by the predetermined additive.

### [1-7. Use]

As the use of the liquid enzyme preparation of the present invention, the liquid enzyme preparation can be used for any application utilizing the enzyme activity. For example, the liquid enzyme preparation of the present invention can be used for the purpose of modifying a protein. The specific embodiment of modifying a protein is not particularly limited. For example, when a protein deamidase is included as the enzyme, the property change of a protein caused by carboxyl groups generated by deamidation of a γ-amide group and a β-amide group of a glutamine residue and an asparagine residue in the protein is utilized. Specifically, examples of the modification of a protein include an increase in solubility of the protein, an increase in water dispersibility, an improvement in emulsifying power, and an improvement in emulsion stability.

Therefore, the liquid enzyme preparation of the present invention can be widely used in the food field. Specifically, examples thereof include use for improving the solubility, dispersibility, and emulsifiability of an animal protein and/or a plant protein in a weakly acidic condition environment, which is in the pH range of normal food (for example, use in the production of coffee whiteners, acidic beverages such as juices, dressings, mayonnaise, and creams); increase in the solubility and dispersibility of poorly soluble plant protein (for example, use in the production of a fried food powder using wheat gluten); use in modifying dough in breadmaking and confectionery (for example, use in the production of crackers, biscuits, cookies, and crust of pizza or pie); use in removing or reducing allergen in allergic protein in food (for example, use in the production of food for wheat allergic patients); use in which the mineral sensitivity of a protein is reduced, the soluble mineral content is increased in a liquid containing the protein and the mineral, and absorbability of the mineral into a human body is enhanced (for example, use in the production of high mineral (e.g. calcium) containing beverages, mineral (e.g. calcium) absorption enhancers); use for reducing bitterness; use for improving the proteolysis rate of a protease, and/or use for enhancing the glutamic acid content (for example, use in the production of amino acid-based seasoning (hydrolyzate of animal protein (HAP), hydrolyzate of plant protein (HVP)), or miso and soy sauce).

### [2. Enzyme activity stabilization method for a liquid enzyme preparation and Protein deamidase activity stabilizer for a liquid enzyme preparation]

As described above for the first liquid enzyme preparation, a liquid enzyme preparation containing an enzyme is packed in a container whose internal space is replaced with an inert gas. Thereby, the enzyme activity stability can be improved. Therefore, the present invention provides: an enzyme activity stabilization method for a liquid enzyme preparation, the method including: packing a liquid enzyme preparation containing an enzyme in a container whose internal space is replaced with an inert gas.

As described above for the second liquid enzyme preparation, the stability of protein deamidase activity can be improved by adding a predetermined additive to a liquid enzyme preparation containing a protein deamidase. Therefore, the present invention provides: a protein deamidase activity stabilizer for a liquid enzyme preparation containing a protein deamidase, the protein deamidase activity stabilizer including an additive selected from the group consisting of sugar alcohol, glycerin, ethanol, sulfite, lecithin, citrate, polyphosphate, tartrate, phytic acid, metaphosphoric acid, sodium chloride, tocotrienol, methionine, pectin, beet dye, paprika dye, rosemary extract, tocopherol, tea extract, and butylhydroxyanisole.

In the enzyme activity stabilization method for a liquid enzyme preparation and the protein deamidase activity stabilizer for a liquid enzyme preparation of the present invention, the type, use amount, and the like of each component are as shown in the column of " 1. Liquid enzyme preparation".

### EXAMPLES

Hereinafter, the present invention will be described with reference to Examples, but the present invention is not limited to these Examples.

Details of each component used in the following test examples are as follows.
- Soy lecithin: lecithin, soybean-derived (FUJIFILM Wako Pure Chemical Corporation)
- Sunflower lecithin: SLP-paste SF (Tsuji Oil Mills co., Ltd.)
- Rapeseed lecithin: SLP-rapeseed paste lecithin preparation (Tsuji Oil Mills co., Ltd.)
- Tocotrienol: Oryza tocotrienol (Oryza Oil & Fat Chemical Co., Ltd.)
- Pectin: sugar beet pectin
- Paprika dye: PapriX powder (GLICO NUTRITION CO., LTD.)
- Rosemary extract: Ethanol-soluble content of ROSEMARY EXTRACT 50%

### (Vidya Herbs Pvt. Ltd.)

- Tocopherol and tea extract: Super emulsion TSM-09 (Taiyo Kagaku Co., Ltd.)
- BHA: butylhydroxyanisole

### [Test Example 1]

A culture solution of the species Chryseobacterium proteolyticum, which is a protein glutaminase-producing strain, was subjected to removal of insoluble components such as contaminant polymers, and then purified by ion exchange chromatography. Thereafter, the pH thereof was adjusted to 6.0 (25°C) using 80% acetic acid or a 6 N aqueous sodium hydroxide solution as a pH adjusting agent. Thus, a liquid enzyme preparation containing protein glutaminase (PG) as a protein deamidase in the concentration shown in Table 1 was prepared. The prepared liquid enzyme preparation was placed in a container, nitrogen was injected from a nitrogen cylinder into the gas phase (the space above the liquid phase made of the liquid enzyme preparation) of the storage container, and taking in and out was repeated 20 times. Thus, oxygen in the container was replaced with nitrogen, and the container was hermetically sealed to obtain a liquid enzyme preparation packed in a container (Example 1-1). Separately, a liquid enzyme preparation packed in a container was obtained in the same manner except that oxygen in the container was not replaced with nitrogen (Comparative Example 1-1).

The liquid enzyme preparation packed in a container thus obtained was left to stand under the storage conditions described in Table 1. The protein deamidase activity value before and after standing was measured by the following method.

Into 1 mL of a 0.2 M phosphate buffer (pH 6.5) containing 30 mM Z-Gln-Gly, 0.1 mL of a sample solution containing a protein deamidase was added. The solution was allowed to stand at 37°C for 10 minutes, and then 1 mL of a 0.4 M TCA solution was added to stop the reaction. As a blank, into 1 mL of a 0.2 M phosphate buffer (pH 6.5) containing 30 mM Z-Gln-Gly, 1 mL of a 0.4 M TCA solution was added, and 0.1 mL of a sample solution containing a protein deamidase was further added. The solution was allowed to stand at 37°C for 10 minutes.

The obtained solution was measured for the amount of ammonia generated in the reaction solution by using AMMONIA TEST WAKO (FUJIFILM Wako Pure Chemical Corporation). The ammonia concentration in the reaction solution was determined from a calibration curve representing the relationship between the ammonia concentration and the absorbance (630 nm), which was prepared using an ammonia standard solution (ammonium chloride).

The protein deamidase activity was calculated from the following formula, assuming that the enzyme amount to generate 1 µmol ammonia per minute is 1 unit (1 U). In the formula, the reaction solution amount is 2.1, the enzyme solution amount is 0.1, and Df is a dilution rate of the enzyme solution. Further, 17.03 is the molecular weight of ammonia. Protein deamidase activity (U/mL) = Ammonia concentration in reaction solution (mg/L) × (1/17.03) × (Reaction solution amount/Enzyme solution amount) × (1/10) × Df

When the protein deamidase activity value before standing was deemed as 100%, the relative value (%) of the protein deamidase activity value after standing was calculated as the residual activity. Table 1 shows the results.

**[Table 1]**

| | | Comparative Example 1-1 | Example 1-1 |
|---|---|---|---|
| PG | | 660 U/ml | |
| pH adjusting agent | | Appropriate amount | Appropriate amount |
| Water | | Remainder | Remainder |
| Total | | 100 w/v% | 100 w/v% |
| pH | | 6 | 6 |
| Nitrogen substitution | | Not performed | Performed |
| Storage condition 40°C/30 days | O₂ concentration in container | 19.7 v/v% | 0.3 v/v% |
| | Dissolved O₂ concentration | 4.84 mg/L | 5.41 mg/L |
| | Residual activity | 41% | 100% |
| Storage condition 25°C/6 months | O₂ concentration in container | 19.9 v/v% | 0.5 v/v% |
| | Dissolved O₂ concentration | 6.48 mg/L | 4.96 mg/L |
| | Residual activity | 25% | 100% |

As shown in the above table, only by packing a liquid enzyme preparation containing a protein deamidase in a container whose internal space was replaced with an inert gas, the activity stability of a protein deamidase was remarkably improved, although the dissolved oxygen concentration was relatively high in the liquid enzyme preparation. When the storage period at 40°C was further extended and the residual activity was measured at 2 months, the residual activity was only 9% in Comparative Example 1-1, whereas the residual activity was successfully maintained at 88% in Example 1-1.

### [Test Example 2]

The residual activity was measured in the same manner as in Test Example 1, except that the composition of the liquid enzyme preparation, the presence or absence of nitrogen substitution in the inner space of the container containing the liquid enzyme preparation, and the storage conditions were as shown in Tables 2A to 2D. The results are shown in Tables 2A to 2D.

**[Table 2A]**

| | Comparative Example 1-2 | Comparative Example 1-3 | Example 1-2 | Comparative Example 1-4 | Example 1-3 | Comparative Example 1-5 | Example 1-4 | Example 1-5 |
|---|---|---|---|---|---|---|---|---|
| PG | 660 U/ml | | | | | | | |
| Sorbitol | 60 | 60 | 60 | 50 | 50 | 50 | 50 | 40 |
| Xylitol | - | - | - | - | - | - | - | - |
| Ethanol | - | - | - | - | - | - | - | - |
| Na sulfite | - | - | - | - | - | - | - | - |
| Soy lecithin | - | - | - | - | - | 0.5 | 0.5 | - |
| Sunflower lecithin | - | - | - | - | - | - | - | 0.1 |
| Rapeseed lecithin | - | - | - | - | - | - | - | - |
| Tri-Na citrate | - | 3 | 3 | 5 | 5 | 1 | 1 | 1 |
| Na polyphosphate | - | - | - | - | - | 0.1 | 0.1 | - |
| Na tartrate | - | - | - | - | - | 5 | 5 | - |
| Phytic acid | - | - | - | - | - | - | - | - |
| Metaphosphoric acid | - | - | - | - | - | - | - | - |
| Na chloride | - | - | - | - | - | - | - | - |
| Tocotrienol | - | - | - | - | - | 0.1 | 0.1 | - |
| Methionine | - | - | - | - | - | 0.5 | 0.5 | - |
| Pectin | | | - | - | - | - | | - |
| Beat dye | - | - | - | - | - | - | - | - |
| Paprika dye | - | - | - | - | - | - | - | - |
| Rosemary extract | - | - | - | - | - | - | - | - |
| Tocopherol and tea extract | - | - | - | - | - | - | - | - |
| BHA | - | - | - | - | - | - | - | - |
| Phosphate buffer | 50 mM | 50 mM | 50 mM | 50 mM | 50 mM | 50 mM | 50 mM | 50 mM |
| Acetate buffer | - | - | - | - | - | - | - | - |
| Citrate buffer | - | - | - | - | - | - | - | - |
| Water | Remainder | Remainder | Remainder | Remainder | Remainder | Remainder | Remainder | Remainder |
| Total | 100 | 100 | 100 | 100 | 100 | 100 | 100 | 100 |
| pH | 6 | 6 | 6 | 6 | 6 | 6 | 6 | 6 |
| Nitrogen substitution | Not performed | Not performed | Performed | Not performed | Performed | Not performed | Performed | Performed |
| O₂ concentration in container (40°C/14 days) | | | 1.8 v/v% | | 1.1 v/v% | | 1.1 v/v% | 0.8 v/v% |
| Residual activity (40°C/14 days) | 73% | 77% | 100% | 76% | 100% | 74% | 100% | 100% |

| | | | | | | | | |
|---|---|---|---|---|---|---|---|---|
| In the table, the unit of the blending amount of the component to which no unit is attached is w/v%. | | | | | | | | |

**[Table 2B]**

| | Comparative Example 1-6 | Example 1-6 | Comparative Example 1-7 | Example 1-7 | Comparative Example 1-8 | Example 1-8 | Comparative Example 1-9 | Example 1-9 | Comparative Example 1-10 | Example 1-10 |
|---|---|---|---|---|---|---|---|---|---|---|
| PG | 660 U/ml | | | | | | | | | |
| Sorbitol | 40 | 40 | 40 | 40 | 30 | 30 | 20 | 20 | 15 | 15 |
| Xylitol | - | - | - | - | - | - | - | - | | - |
| Ethanol | - | - | - | - | - | - | - | - | - | - |
| Na sulfite | - | - | - | - | - | - | - | - | - | - |
| Soy lecithin | - | - | - | - | - | - | - | - | - | - |
| Sunflower lecithin | - | - | - | - | - | - | - | - | - | - |
| Rapeseed lecithin | - | - | - | - | - | - | - | - | - | - |
| Tri-Na citrate | 1 | 1 | 3 | 3 | 10 | 10 | 10 | 10 | 10 | 10 |
| Na polyphosphate | - | - | - | - | - | - | - | - | - | - |
| Na tartrate | - | - | - | - | - | - | - | - | - | - |
| Phytic acid | - | - | - | - | - | - | - | - | - | - |
| Metaphosphoric acid | - | - | - | - | - | - | - | - | - | - |
| Na chloride | - | - | - | - | - | - | - | - | - | - |
| Tocotrienol | - | - | - | - | - | - | - | - | - | - |
| Methionine | - | - | - | - | - | - | - | - | - | - |
| Pectin | - | - | - | - | - | - | - | - | - | - |
| Beat dye | - | - | - | - | - | - | - | - | - | - |
| Paprika dye | - | - | - | - | - | - | - | - | - | - |
| Rosemary extract | - | - | - | - | - | - | - | - | - | - |
| Tocopherol and tea extract | - | - | - | - | - | - | - | - | - | - |
| BHA | - | - | - | - | - | - | - | - | | - |
| Phosphate buffer | - | - | | - | - | - | | - | | - |
| Acetate buffer | 50 mM | 50 mM | 50 mM | 50 mM | 50 mM | 50 mM | 50 mM | 50 mM | 50 mM | 50 mM |
| Citrate buffer | - | - | - | - | - | | | - | - | - |
| Water | Remainder | Remainder | Remainder | Remainder | Remainder | Remainder | Remainder | Remainder | Remainder | Remainder |
| Total | 100 | 100 | 100 | 100 | 100 | 100 | 100 | 100 | 100 | 100 |
| pH | 6 | 6 | 6 | 6 | 6 | 6 | 6 | 6 | 6 | 6 |
| Nitrogen substitution | Not performed | Performed | Not performed | Performed | Not performed | Performed | Not performed | Performed | Not performed | Performed |
| O₂ concentration in container (40°C/14 days) | | 0.9 v/v% | | 0.9 v/v% | | 0.9 v/v% | | 0.9 v/v% | | 0.9 v/v% |
| Residual activity (40°C/14 days) | 73% | 95% | 71% | 97% | | 100% | 74% | 99% | 74% | 96% |
| O₂ concentration in container (25°C/3 months) | | 1.2 v/v% | | 1.2 v/v% | | 1.2 v/v% | | 1.2 v/v% | | 1.2 v/v% |
| Residual activity (25°C/3 months) | 73% | 96% | 74% | 97% | 73% | 98% | 72% | 98% | 70% | 99% |

| | | | | | | | | | | |
|---|---|---|---|---|---|---|---|---|---|---|
| In the table, the unit of the blending amount of the component to which no unit is attached is w/v%. | | | | | | | | | | |

**[Table 2C]**

| | Comparative Example 1-11 | Example 1-11 | Comparative Example 1-12 | Example 1-12 | Comparative Example 1-13 | Example 1-13 | Comparative Example 1-14 | Example 1-14 | Comparative Example 1-15 | Example 1-15 |
|---|---|---|---|---|---|---|---|---|---|---|
| PG | 660 U/ml | | | | | | | | | |
| Sorbitol | 20 | 20 | 15 | 15 | - | - | - | - | - | - |
| Xylitol | - | - | - | - | - | - | - | - | | - |
| Ethanol | 5 | 5 | 5 | 5 | 10 | 10 | 10 | 10 | - | - |
| Na sulfite | - | - | - | - | - | - | - | - | - | - |
| Soy lecithin | - | - | - | - | - | - | - | - | - | - |
| Sunflower lecithin | - | - | - | - | - | - | - | - | - | - |
| Rapeseed lecithin | - | - | - | - | - | - | - | - | - | - |
| Tri-Na citrate | 5 | 5 | 5 | 5 | - | - | 5 | 5 | 10 | 10 |
| Na polyphosphate | - | - | - | - | - | - | - | - | - | - |
| Na tartrate | - | - | - | - | - | - | - | - | - | - |
| Phytic acid | - | - | - | - | - | - | - | - | - | - |
| Metaphosphoric acid | - | - | - | - | - | - | - | - | - | - |
| Na chloride | 5 | 5 | 5 | 5 | - | - | - | - | - | - |
| Tocotrienol | - | - | - | - | - | - | - | - | - | - |
| Methionine | - | - | - | - | - | - | - | - | - | - |
| Pectin | - | - | - | - | - | - | - | - | - | - |
| Beat dye | - | - | - | - | - | - | - | - | - | - |
| Paprika dye | - | - | - | - | - | - | - | - | - | - |
| Rosemary extract | - | - | - | - | - | - | - | - | - | - |
| Tocopherol and tea extract | - | - | - | - | - | - | - | - | - | - |
| BHA | - | - | - | - | - | - | - | - | - | - |
| Phosphate buffer | - | - | - | - | - | - | - | - | - | - |
| Acetate buffer | 50 mM | 50 mM | 50 mM | 50 mM | 50 mM | 50 mM | 50 mM | 50 mM | 50 mM | 50 mM |
| Citrate buffer | - | - | - | - | - | - | - | - | - | - |
| Water | Remainder | Remainder | Remainder | Remainder | Remainder | Remainder | Remainder | Remainder | Remainder | Remainder |
| Total | 100 | 100 | 100 | 100 | 100 | 100 | 100 | 100 | 100 | 100 |
| pH | 6 | 6 | 6 | 6 | 6 | 6 | 6 | 6 | 6 | 6 |
| Nitrogen substitution | Not performed | Performed | Not performed | Performed | Not performed | Performed | Not performed | Performed | Not performed | Performed |
| O₂ concentration in container (40°C/14 days) | | 0.9 v/v% | | 0.9 v/v% | | 0.9 v/v% | | 0.9 v/v% | | 0.9 v/v% |
| Residual activity (40°C/14 days) | 57% | 97% | 58% | 100% | 73% | 98% | 57% | 99% | 75% | 100% |
| O₂ concentration in container (25°C/3 months) | | | | | | 1.2 v/v% | | 1.2 v/v% | | 1.2 v/v% |
| Residual activity (25°C/3 months) | | | | | 63% | 100% | 66% | 100% | 77% | 100% |

| | | | | | | | | | | |
|---|---|---|---|---|---|---|---|---|---|---|
| In the table, the unit of the blending amount of the component to which no unit is attached is w/v%. | | | | | | | | | | |

**[Table 2D]**

| | Comparative Example 1-16 | Example 1-16 | Comparative Example 1-17 | Example 1-17 |
|---|---|---|---|---|
| PG | 660 U/ml | | | |
| Sorbitol | - | - | - | - |
| Xylitol | - | - | - | - |
| Ethanol | - | - | - | - |
| Na sulfite | | - | | - |
| Soy lecithin | - | - | - | - |
| Sunflower lecithin | - | - | - | - |
| Rapeseed lecithin | - | - | - | - |
| Tri-Na citrate | - | - | 5 | 5 |
| Na polyphosphate | - | - | - | - |
| Na tartrate | - | - | - | - |
| Phytic acid | - | - | - | - |
| Metaphosphoric acid | - | - | - | - |
| Na chloride | 15 | 15 | 15 | 15 |
| Tocotrienol | - | - | - | - |
| Methionine | - | - | - | - |
| Pectin | | - | | - |
| Beat dye | - | - | - | - |
| Paprika dye | - | - | - | - |
| Rosemary extract | - | - | - | - |
| Tocopherol and tea extract | - | - | - | - |
| BHA | - | - | - | - |
| Phosphate buffer | - | - | - | - |
| Acetate buffer | 50 mM | 50 mM | 50 mM | 50 mM |
| Citrate buffer | - | - | - | - |
| Water | Remainder | Remainder | Remainder | Remainder |
| Total | 100 | 100 | 100 | 100 |
| pH | 6 | 6 | 6 | 6 |
| Nitrogen substitution | Not performed | Performed | Not performed | Performed |
| O₂ concentration in container (25°C/3 months) | | 1.2 v/v% | | 1.2 v/v% |
| Residual activity (25°C/3 months) | 46% | 99% | 42% | 96% |

| | | | | |
|---|---|---|---|---|
| In the table, the unit of the blending amount of the component to which no unit is attached is w/v%. | | | | |

As shown in the above tables, a liquid enzyme preparation containing a protein deamidase and packed in a container whose internal space is replaced with an inert gas exhibited a remarkably high enzyme activity stability.

### [Test Example 3]

A culture solution of the species Streptomyces mobaraensis, which is a transglutaminase-producing strain, was subjected to removal of insoluble components such as contaminant polymers. Thereafter, the pH thereof was adjusted to 6.0 (25°C) using 80% acetic acid or a 6 N aqueous sodium hydroxide solution as a pH adjusting agent. Thus, a liquid enzyme preparation containing a transglutaminase in the concentration shown in Table 3 was prepared. The prepared liquid enzyme preparation was placed in a container, nitrogen was injected from a nitrogen cylinder into the gas phase (the space above the liquid phase made of the liquid enzyme preparation) of the storage container, and taking in and out was repeated 20 times. Thus, the container had oxygen replaced with nitrogen, and hermetically sealed to obtain a liquid enzyme preparation packed in a container (Examples 1-18 to 1-21). Separately, a liquid enzyme preparation packed in a container was obtained in the same manner except that oxygen in the container was not replaced with nitrogen (Comparative Examples 1-18 to 1-21).

The liquid enzyme preparation packed in a container thus obtained was left to stand at 25°C for 6 months. The transglutaminase activity value before and after standing was measured by the following method.

### <Method for measuring transglutaminase activity>

The enzyme activity of transglutaminase was measured by the method described below using benzyloxycarbonyl-L-glutaminylglycine and hydroxylamine as a substrate.

A substrate solution was prepared by dissolving 2.42 g of 2-amino-2-hydroxymethyl-1.3-propanediol, 0.70 g of hydroxyammonium hydrochloride, 0.31 g of reduced glutathione, and 1.01 g of Z-Gln-Gly (benzyloxycarbonyl-L-glutaminylglycine) in distilled water, and setting the total amount thereof to 100 mL (pH 6.0).

A coloring solution was prepared by mixing 30 mL of a 3 M hydrochloric acid solution, 30 mL of a 12 wt% trichloroacetic acid solution, and 30 mL of a 5 wt% iron (III) chloride solution.

A sample solution was prepared by diluting the enzyme with a 200 mM MES buffer (pH 6.0) into an appropriate concentration.

The reaction was performed at 37°C for 10 minutes after adding and mixing 100 µL of the substrate solution into 10 µL of the sample solution. The reaction was stopped by adding 100 µL of the coloring solution and an Fe complex was formed, and then the absorbance at 525 nm was measured. As a control, a previously heat-inactivated sample solution was reacted in the same manner and measured for absorbance, thereby determining the absorbance difference from the non-heat-inactivated sample solution. Separately, a calibration curve was prepared using L-glutamic acid-γ-monohydroxamic acid instead of the sample solution, and the amount of generated hydroxamic acid was determined from the absorbance difference. The enzyme activity to generate 1 µmol hydroxamic acid for 1 minute was defined as 1 unit (1 U).

When the transglutaminase activity value before standing was deemed as 100%, the relative value (%) of the transglutaminase activity value after standing was calculated as the residual activity. Table 3 shows the results.

**[Table 3]**

| | Comparative Example 1-18 | Example 1-18 | Comparative Example 1-19 | Example 1-19 | Comparative Example 1-20 | Example 1-20 | Comparative Example 1-21 | Example 1-21 |
|---|---|---|---|---|---|---|---|---|
| Transglutaminase | 100 U/ml | | 200 U/ml | | 400 U/mL | | 500 U/mL | |
| pH adjusting agent | Appropriate amount | Appropriate amount | Appropriate amount | Appropriate amount | Appropriate amount | Appropriate amount | Appropriate amount | Appropriate amount |
| Water | Remainder | Remainder | Remainder | Remainder | Remainder | Remainder | Remainder | Remainder |
| Total | 100 w/v% | 100 w/v% | 100 w/v% | 100 w/v% | 100 w/v% | 100 w/v% | 100 w/v% | 100 w/v% |
| pH | 6 | 6 | 6 | 6 | 6 | 6 | 6 | 6 |
| Nitrogen substitution | Not performed | Performed | Not performed | Performed | Not performed | Performed | Not performed | Performed |
| O₂ concentration in container (25°C/6 months) | | 0.4 v/v% | | 0.4 v/v% | | 0.4 v/v% | | 0.4 v/v% |
| Residual activity (25°C/6 months) | 65% | 69% | 50% | 65% | 47% | 64% | 39% | 59% |

As shown in Table 3, only by packing a liquid enzyme preparation containing a transglutaminase in a container whose internal space was replaced with an inert gas, the activity stability of a transglutaminase was improved.

### [Test Example 4]

The residual activity was measured in the same manner as in Test Example 3, except that the composition of the liquid enzyme preparation and the presence or absence of nitrogen substitution in the inner space of the container packing the liquid enzyme preparation were as shown in Tables 4A to 4D. The results are shown in Tables 4A to 4D.

**[Table 4A]**

| | Comparative Example 1-22 | Example 1-22 | Comparative Example 1-23 | Example 1-23 | Comparative Example 1-24 | Example 1-24 | Comparative Example 1-25 | Example 1-25 |
|---|---|---|---|---|---|---|---|---|
| Transglutaminase | 100 U/mL | | 200 U/mL | | 400 U/mL | | 500 U/mL | |
| Sorbitol | 40 | 40 | 40 | 40 | 40 | 40 | 40 | 40 |
| Glycerin | - | - | - | - | - | - | - | - |
| Maltitol | - | - | - | - | - | - | - | - |
| Xylitol | - | - | - | - | - | - | - | - |
| Tri-Na citrate | - | - | - | - | - | - | - | - |
| Acetate buffer | 50 mM | 50 mM | 50 mM | 50 mM | 50 mM | 50 mM | 50 mM | 50 mM |
| Water | Remainder | Remainder | Remainder | Remainder | Remainder | Remainder | Remainder | Remainder |
| Total | 100 | 100 | 100 | 100 | 100 | 100 | 100 | 100 |
| pH | 6 | 6 | 6 | 6 | 6 | 6 | 6 | 6 |
| Nitrogen substitution | Not performed | Performed | Not performed | Performed | Not performed | Performed | Not performed | Performed |
| O₂ concentration in container (25°C/6 months) | | 0.6 v/v% | | 0.6 v/v% | | 0.6 v/v% | | 0.6 v/v% |
| Residual activity (25°C/6 months) | 29% | 32% | 32% | 41% | 42% | 56% | 41% | 58% |

| | | | | | | | | |
|---|---|---|---|---|---|---|---|---|
| In the table, the unit of the blending amount of the component to which no unit is attached is w/v%. | | | | | | | | |

**[Table 4B]**

| | Comparative Example 1-26 | Example 1-26 | Comparative Example 1-27 | Example 1-27 | Comparative Example 1-28 | Example 1-28 | Comparative Example 1-29 | Example 1-29 |
|---|---|---|---|---|---|---|---|---|
| Transglutaminase | 100 U/mL | | 200 U/mL | | 400 U/mL | | 500 U/mL | |
| Sorbitol | 40 | 40 | 40 | 40 | 40 | 40 | 40 | 40 |
| Glycerin | - | - | - | - | - | - | - | - |
| Maltitol | - | - | - | - | - | - | - | - |
| Xylitol | - | - | - | - | - | - | - | - |
| Tri-Na citrate | 3 | 3 | 3 | 3 | 3 | 3 | 3 | 3 |
| Acetate buffer | 50 mM | 50 mM | 50 mM | 50 mM | 50 mM | 50 mM | 50 mM | 50 mM |
| Water | Remainder | Remainder | Remainder | Remainder | Remainder | Remainder | Remainder | Remainder |
| Total | 100 | 100 | 100 | 100 | 100 | 100 | 100 | 100 |
| pH | 6 | 6 | 6 | 6 | 6 | 6 | 6 | 6 |
| Nitrogen substitution | Not performed | Performed | Not performed | Performed | Not performed | Performed | Not performed | Performed |
| O₂ concentration in container (25°C/6 months) | | 0.6 v/v% | | 0.6 v/v% | | 0.6 v/v% | | 0.6 v/v% |
| Residual activity (25°C/6 months) | 26% | 30% | 31% | 42% | 44% | 62% | 45% | 65% |

| | | | | | | | | |
|---|---|---|---|---|---|---|---|---|
| In the table, the unit of the blending amount of the component to which no unit is attached is w/v%. | | | | | | | | |

**[Table 4C]**

| | Comparative Example 1-30 | Example 1-30 | Comparative Example 1-31 | Example 1-31 | Comparative Example 1-32 | Example 1-32 |
|---|---|---|---|---|---|---|
| Transglutaminase | 200 U/mL | | 400 U/mL | | 500 U/mL | |
| Sorbitol | - | - | - | - | - | - |
| Glycerin | 40 | 40 | 40 | 40 | 40 | 40 |
| Maltitol | - | - | - | - | - | - |
| Xylitol | - | - | - | - | - | - |
| Tri-Na citrate | - | - | - | - | - | - |
| Acetate buffer | 50 mM | 50 mM | 50 mM | 50 mM | 50 mM | 50 mM |
| Water | Remainder | Remainder | Remainder | Remainder | Remainder | Remainder |
| Total | 100 | 100 | 100 | 100 | 100 | 100 |
| pH | 6 | 6 | 6 | 6 | 6 | 6 |
| Nitrogen substitution | Not performed | Performed | Not performed | Performed | Not performed | Performed |
| O₂ concentration in container (25°C/6 months) | | 0.4 v/v% | | 0.4 v/v% | | 0.4 v/v% |
| Residual activity (25°C/6 months) | 57% | 61% | 66% | 74% | 56% | 71% |

| | | | | | | |
|---|---|---|---|---|---|---|
| In the table, the unit of the blending amount of the component to which no unit is attached is w/v%. | | | | | | |

**[Table 4D]**

| | Comparative Example 1-33 | Example 1-33 | Comparative Example 1-34 | Example 1-34 | Comparative Example 1-35 | Example 1-35 | Comparative Example 1-36 | Example 1-36 | Comparative Example 1-37 | Example 1-37 |
|---|---|---|---|---|---|---|---|---|---|---|
| Transglutaminase | 100 U/mL | | 200 U/mL | | 400 U/mL | | 500 U/mL | | 500 U/mL | |
| Sorbitol | - | - | - | - | - | - | - | - | - | - |
| Glycerin | - | - | - | - | - | - | - | - | - | - |
| Maltitol | 40 | 40 | 40 | 40 | 40 | 40 | 40 | 40 | - | - |
| Xylitol | - | - | - | - | - | - | - | - | 40 | 40 |
| Tri-Na citrate | - | - | - | - | - | - | - | - | - | - |
| Acetate buffer | 50 mM | 50 mM | 50 mM | 50 mM | 50 mM | 50 mM | 50 mM | 50 mM | 50 mM | 50 mM |
| Water | Remainder | Remainder | Remainder | Remainder | Remainder | Remainder | Remainder | Remainder | Remainder | Remainder |
| Total | 100 | 100 | 100 | 100 | 100 | 100 | 100 | 100 | 100 | 100 |
| pH | 6 | 6 | 6 | 6 | 6 | 6 | 6 | 6 | 6 | 6 |
| Nitrogen substitution | Not performed | Performed | Not performed | Performed | Not performed | Performed | Not performed | Performed | Not performed | Performed |
| O₂ concentration in container (25°C/6 months) | | 0.4 v/v% | | 0.4 v/v% | | 0.4 v/v% | | 0.4 v/v% | | 0.4 v/v% |
| Residual activity (25°C/6 months) | 73% | 84% | 73% | 86% | 60% | 78% | 52% | 78% | 49% | 66% |

| | | | | | | | | | | |
|---|---|---|---|---|---|---|---|---|---|---|
| In the table, the unit of the blending amount of the component to which no unit is attached is w/v%. | | | | | | | | | | |

As shown in Tables 4A to 4D, by packing a liquid enzyme preparation containing a transglutaminase in a container whose internal space is replaced with an inert gas, the enzyme activity stability was improved.

### [Test Example 5]

The residual activity was measured in the same manner as in Test Example 1, except that the composition of the liquid enzyme preparation, the presence or absence of nitrogen substitution in the inner space of the container packing the liquid enzyme preparation, and the storage conditions were as shown in Tables 5A to 5D. The results are shown in Tables 5A to 5D.

**[Table 5A]**

| | Comparative Example 2-1 | Example 2-1 | Example 2-2 | Example 2-3 | Example 2-4 | Example 2-5 | Example 2-6 | Example 2-7 | Example 2-8 | Example 2-9 | Example 2-10 |
|---|---|---|---|---|---|---|---|---|---|---|---|
| PG | 660 U/ml | | | | | | | | | | |
| Sorbitol | - | 60 | 70 | - | 25 | 40 | 60 | 60 | 60 | 60 | 60 |
| Xylitol | - | - | - | 50 | 25 | - | - | - | - | - | - |
| Ethanol | - | - | - | - | - | - | - | - | - | - | - |
| Na sulfite | - | - | - | - | - | 0.2 | - | - | - | - | - |
| Soy lecithin | - | - | - | - | - | - | - | - | - | - | - |
| Sunflower lecithin | - | - | - | - | - | - | - | - | - | - | - |
| Rapeseed lecithin | - | - | - | - | - | - | - | - | - | - | - |
| Tri-Na citrate | - | - | - | - | - | - | - | - | - | - | - |
| Na polyphosphate | - | - | - | - | - | - | - | - | - | - | - |
| Na tartrate | - | - | - | - | - | - | - | - | - | - | - |
| Phytic acid | - | - | - | - | - | - | - | - | - | - | - |
| Metaphosphoric acid | - | - | - | - | - | - | - | - | - | - | - |
| Na chloride | - | - | - | - | - | - | - | - | - | - | - |
| Tocotrienol | - | - | - | - | - | - | - | - | - | - | - |
| Methionine | - | - | - | - | - | - | - | - | - | - | - |
| Pectin | - | - | - | - | - | - | - | 0.05 | - | - | - |
| Beat dye | - | - | - | - | - | - | - | - | 0.05 | - | - |
| Paprika dye | - | - | - | - | - | - | 0.1 | - | - | - | - |
| Rosemary extract | - | - | - | - | - | - | - | - | - | 0.05 | 0.5 |
| Tocopherol and tea extract | - | - | - | - | - | - | - | - | - | - | - |
| BHA | - | - | - | - | - | - | - | - | - | - | - |
| Phosphate buffer | - | 50 mM | 50 mM | 50 mM | 50 mM | - | 50 mM | 50 mM | 50 mM | 50 mM | 50 mM |
| Acetate buffer | - | - | - | - | - | - | - | - | - | - | - |
| Citrate buffer | - | - | - | - | - | 10 mM | - | - | - | - | - |
| Water | Remainder | Remainder | Remainder | Remainder | Remainder | Remainder | Remainder | Remainder | Remainder | Remainder | Remainder |
| Total | 100 | 100 | 100 | 100 | 100 | 100 | 100 | 100 | 100 | 100 | 100 |
| pH | 6 | 6 | 6 | 6 | 6 | 6 | 6 | 6 | 6 | 6 | 6 |
| Nitrogen substitution | Not performed | Not performed | Not performed | Not performed | Not performed | Not performed | Not performed | Not performed | Not performed | Not performed | Not performed |
| Residual activity (40°C/14 days) | 63% | 73% | 79% | 73% | 73% | 75% | 68% | 70% | 65% | 74% | 70% |

| | | | | | | | | | | | |
|---|---|---|---|---|---|---|---|---|---|---|---|
| In the table, the unit of the blending amount of the component to which no unit is attached is w/v%. | | | | | | | | | | | |

**[Table 5B]**

| | Comparative Example 2-1 | Example 2-11 | Example 2-12 | Example 2-13 | Example 2-14 | Example 2-15 | Example 2-16 | Example 2-17 | Example 2-18 | Example 2-19 |
|---|---|---|---|---|---|---|---|---|---|---|
| PG | 660 U/ml | | | | | | | | | |
| Sorbitol | - | 60 | 60 | 60 | 60 | 60 | 60 | 60 | 60 | 60 |
| Xylitol | - | - | - | - | - | - | - | - | - | - |
| Ethanol | - | - | - | - | - | - | - | - | - | - |
| Na sulfite | - | - | - | - | - | - | - | - | - | - |
| Soy lecithin | - | - | - | - | - | - | - | - | - | - |
| Sunflower lecithin | - | - | - | - | - | - | - | - | - | - |
| Rapeseed lecithin | - | 0.1 | 1 | 0.1 | 1 | - | - | - | - | - |
| Tri-Na citrate | - | - | - | - | - | - | - | - | - | - |
| Na polyphosphate | - | - | - | - | - | - | - | - | - | - |
| Na tartrate | - | - | - | - | - | - | - | - | - | - |
| Phytic acid | - | - | - | - | - | - | 0.1 | 1 | - | - |
| Metaphosphoric acid | - | - | - | - | - | - | - | - | 0.1 | 1 |
| Na chloride | - | - | - | - | - | - | - | - | - | - |
| Tocotrienol | - | 0.1 | 0.1 | 1 | 1 | - | - | - | - | - |
| Methionine | - | - | - | - | - | - | - | - | - | - |
| Pectin | - | - | - | - | - | - | - | - | - | - |
| Beat dye | - | - | - | - | - | - | - | - | - | - |
| Paprika dye | - | - | - | - | - | - | - | - | - | - |
| Rosemary extract | - | - | - | - | - | - | - | - | - | - |
| Tocopherol and tea extract | - | - | - | - | - | 0.1 | - | - | - | - |
| BHA | - | - | - | - | - | - | - | - | - | - |
| Phosphate buffer | - | 50 mM | 50 mM | 50 mM | 50 mM | 50 mM | 50 mM | 50 mM | 50 mM | 50 mM |
| Acetate buffer | - | - | - | - | - | - | - | - | - | - |
| Citrate buffer | - | - | - | - | - | - | - | - | - | - |
| Water | Remainder | Remainder | Remainder | Remainder | Remainder | Remainder | Remainder | Remainder | Remainder | Remainder |
| Total | 100 | 100 | 100 | 100 | 100 | 100 | 100 | 100 | 100 | 100 |
| pH | 6 | 6 | 6 | 6 | 6 | 6 | 6 | 6 | 6 | 6 |
| Nitrogen substitution | Not performed | Not performed | Not performed | Not performed | Not performed | Not performed | Not performed | Not performed | Not performed | Not performed |
| Residual activity (40°C/14 days) | 63% | 83% | 76% | 78% | 78% | 73% | 80% | 77% | 73% | 69% |

| | | | | | | | | | | |
|---|---|---|---|---|---|---|---|---|---|---|
| In the table, the unit of the blending amount of the component to which no unit is attached is w/v%. | | | | | | | | | | |

**[Table 5C]**

| | Comparative Example 2-1 | Example 2-20 | Example 2-21 | Example 2-22 | Example 2-23 | Example 2-24 | Example 2-25 | Example 2-26 | Example 2-27 |
|---|---|---|---|---|---|---|---|---|---|
| PG | 660 U/ml | | | | | | | | |
| Sorbitol | - | 60 | 60 | 60 | 60 | 50 | 50 | 60 | 60 |
| Xylitol | - | - | - | - | - | - | - | - | - |
| Ethanol | - | - | - | - | - | - | - | - | - |
| Na sulfite | - | - | - | - | - | - | - | - | - |
| Soy lecithin | - | - | - | - | - | - | 0.5 | - | - |
| Sunflower lecithin | - | - | - | - | - | - | - | - | - |
| Rapeseed lecithin | - | - | - | - | - | - | - | 0.1 | 1 |
| Tri-Na citrate | - | - | - | - | 3 | 5 | 1 | - | - |
| Na polyphosphate | - | - | 0.1 | 1 | - | - | 0.1 | - | - |
| Na tartrate | - | - | - | - | - | - | 5 | - | - |
| Phytic acid | - | - | - | - | - | - | - | - | - |
| Metaphosphoric acid | - | - | - | - | - | - | - | - | - |
| Na chloride | - | - | - | - | - | - | - | - | - |
| Tocotrienol | - | - | - | - | - | - | 0.1 | - | - |
| Methionine | - | - | - | - | - | - | 0.5 | - | - |
| Pectin | - | - | - | - | - | - | - | - | - |
| Beat dye | - | - | - | - | - | - | - | - | - |
| Paprika dye | - | - | - | - | - | - | - | - | - |
| Rosemary extract | - | - | - | - | - | - | - | - | - |
| Tocopherol and te extract | - | - | - | - | - | - | - | - | - |
| BHA | - | 0.1 | - | - | - | - | - | - | - |
| Phosphate buffer | - | 50 mM | 50 mM | 50 mM | 50 mM | 50 mM | 50 mM | 50 mM | 50 mM |
| Acetate buffer | - | - | - | - | - | - | - | - | - |
| Citrate buffer | - | - | - | - | - | - | - | - | - |
| Water | Remainder | Remainder | Remainder | Remainder | Remainder | Remainder | Remainder | Remainder | Remainder |
| Total | 100 | 100 | 100 | 100 | 100 | 100 | 100 | 100 | 100 |
| pH | 6 | 6 | 6 | 6 | 6 | 6 | 6 | 6 | 6 |
| Nitrogen substitution | Not performed | Not performed | Not performed | Not performed | Not performed | Not performed | Not performed | Not performed | Not performed |
| Residual activity (40°C/14 days) | 63% | 79% | 69% | 80% | 77% | 76% | 74% | 77% | 75% |

| | | | | | | | | | |
|---|---|---|---|---|---|---|---|---|---|
| In the table, the unit of the blending amount of the component to which no unit is attached is w/v%. | | | | | | | | | |

**[Table 5D]**

| | Comparative Example 2-1 | Example 2-28 | Example 2-29 | Example 2-30 | Example 2-31 | Example 2-32 | Example 2-33 | Example 2-34 | Example 2-35 | Example 2-36 |
|---|---|---|---|---|---|---|---|---|---|---|
| PG | 660 U/ml | | | | | | | | | |
| Sorbitol | - | - | - | - | 15 | 20 | 30 | 40 | 40 | 60 |
| Xylitol | - | - | - | - | - | - | - | - | - | - |
| Ethanol | - | 10 | 10 | - | - | - | - | - | - | - |
| Na sulfite | - | - | - | - | - | - | - | - | - | 0.2 |
| Soy lecithin | - | - | - | - | - | - | - | - | - | - |
| Sunflower lecithin | - | - | - | - | - | - | - | - | - | - |
| Rapeseed lecithin | - | - | - | - | - | - | - | - | - | - |
| Tri-Na citrate | - | - | 5 | 10 | 10 | 10 | 10 | 1 | 3 | - |
| Na polyphosphate | - | - | - | - | - | - | - | - | - | - |
| Na tartrate | - | - | - | - | - | - | - | - | - | - |
| Phytic acid | - | - | - | - | - | - | - | - | - | - |
| Metaphosphoric acid | - | - | - | - | - | - | - | - | - | - |
| Na chloride | - | - | - | - | - | - | - | - | - | - |
| Tocotrienol | - | - | - | - | - | - | - | - | - | - |
| Methionine | - | - | - | - | - | - | - | - | - | - |
| Pectin | - | - | - | - | - | - | - | - | - | - |
| Beat dye | - | - | - | - | - | - | - | - | - | - |
| Paprika dye | - | - | - | - | - | - | - | - | - | - |
| Rosemary extract | - | - | - | - | - | - | - | - | - | - |
| Tocopherol and tea extract | - | - | - | - | - | - | - | - | - | - |
| BHA | - | - | - | - | - | - | - | - | - | - |
| Phosphate buffer | - | - | - | - | - | - | - | - | - | 50 mM |
| Acetate buffer | - | 50 mM | 50 mM | 50 mM | 50 mM | 50 mM | 50 mM | 50 mM | 50 mM | - |
| Citrate buffer | - | - | - | - | - | - | - | - | - | - |
| Water | Remainder | Remainder | Remainder | Remainder | Remainder | Remainder | Remainder | Remainder | Remainder | Remainder |
| Total | 100 | 100 | 100 | 100 | 100 | 100 | 100 | 100 | 100 | 100 |
| pH | 6 | 6 | 6 | 6 | 6 | 6 | 6 | 6 | 6 | 6 |
| Nitrogen substitution | Not performed | Not performed | Not performed | Not performed | Not performed | Not performed | Not performed | Not performed | Not performed | Not performed |
| Residual activity (40°C/14 days) | 63% | 73% | | 75% | 74% | 74% | | 73% | | 93% |
| Residual activity (25°C/3 months) | 61% | 63% | 66% | 77% | 70% | 72% | 73% | 73% | 74% | 98% |

| | | | | | | | | | | |
|---|---|---|---|---|---|---|---|---|---|---|
| In the table, the unit of the blending amount of the component to which no unit is attached is w/v%. | | | | | | | | | | |

As shown in Tables 5A to 5D, the activity stability of a protein deamidase was improved by adding a predetermined additive to a liquid enzyme preparation containing the protein deamidase. Many of these predetermined additives are components known as an antioxidant. However, the effect of improving the protein deamidase activity stability was not obtained when many other components that have been known as an antioxidant, such as ascorbic acid, were used. In view of the fact, it was found that the effect of improving the protein deamidase activity stability was a specific effect that is obtained by using the predetermined components.

## Claims

1. A liquid enzyme preparation comprising an enzyme, wherein the liquid enzyme preparation is packed in a container whose internal space is replaced with an inert gas.

2. The liquid enzyme preparation according to claim 1, wherein the enzyme is a protein deamidase or a transglutaminase.

3. The liquid enzyme preparation according to claim 1, wherein the enzyme activity is 0.1 to 10,000 U/ml.

4. The liquid enzyme preparation according to claim 1, comprising an additive selected from the group consisting of sugar alcohol, glycerin, ethanol, sulfite, lecithin, citrate, polyphosphate, tartrate, phytic acid, metaphosphoric acid, sodium chloride, tocotrienol, methionine, pectin, beet dye, paprika dye, rosemary extract, tocopherol, tea extract, and butylhydroxyanisole.

5. A liquid enzyme preparation comprising: a protein deamidase; and an additive selected from the group consisting of sugar alcohol, glycerin, ethanol, sulfite, lecithin, citrate, polyphosphate, tartrate, phytic acid, metaphosphoric acid, sodium chloride, tocotrienol, methionine, pectin, beet dye, paprika dye, rosemary extract, tocopherol, tea extract, and butylhydroxyanisole.

6. The liquid enzyme preparation according to claim 4 or 5, wherein the additive is contained in a total amount of 5 w/v% or more.

7. The liquid enzyme preparation according to claim 4 or 5, wherein the additive is contained in a total amount of 80 w/v% or less.

8. The liquid enzyme preparation according to claim 1 or 5, having a pH of 5.5 or more.

9. The liquid enzyme preparation according to claim 1 or 5, having a pH of 8.2 or less.

10. The liquid enzyme preparation according to claim 5, wherein the protein deamidase activity is 0.1 to 10,000 U/ml.

11. The liquid enzyme preparation according to claim 2 or 5, wherein the protein deamidase is derived from Chryseobacterium proteolyticum.

12. An enzyme activity stabilization method for a liquid enzyme preparation, the method comprising: packing a liquid enzyme preparation containing an enzyme in a container whose internal space is replaced with an inert gas.

13. A protein deamidase activity stabilizer for a liquid enzyme preparation containing a protein deamidase, the protein deamidase activity stabilizer comprising an additive selected from the group consisting of sugar alcohol, glycerin, ethanol, sulfite, lecithin, citrate, polyphosphate, tartrate, phytic acid, metaphosphoric acid, sodium chloride, tocotrienol, methionine, pectin, beet dye, paprika dye, rosemary extract, tocopherol, tea extract, and butylhydroxyanisole.
